# EUROPEAN PATENT SPECIFICATION

(11) **EP 3 542 856 B1**
(45) Date of publication and mention of the grant of the patent: **28.07.2021**
(21) Application number: 17872516.4
(22) Date of filing: 29.09.2017
(51) Int. Cl.: A61N 5/06, A61N 1/36, A61N 1/08, G01J 3/44, G01N 21/552

(54) **DEEP BRAIN STIMULATION APPARATUS ON BASIS OF SURFACE-ENHANCED RAMAN SPECTROSCOPY**
VORRICHTUNG ZUR TIEFEN HIRNSTIMULATION AUF DER BASIS VON OBERFLÄCHENVERSTÄRKTER RAMANSPEKTROSKOPIE
APPAREIL DE STIMULATION CÉRÉBRALE PROFONDE SUR LA BASE D'UNE SPECTROSCOPIE RAMAN EXALTÉE DE SURFACE

(30) Priority: 15.11.2016 KR 20160152243
(43) Date of publication of application: 25.09.2019
(73) Proprietor: Samsung Life Public Welfare Foundation, Seoul 04348 (KR)
(72) Inventor: PARK, Eun Kyoung, Seoul 06351 (KR); KANG, Min Hee, Seoul 06351 (KR); KWON, Soon Young, Seoul 06351 (KR); LEE, Kyu-Sung, Seoul 06351 (KR)
(74) Representative: Patentanwälte Olbricht Buchhold Keulertz
(86) International application number: PCT/KR2017/010921
(87) International publication number: WO 2018/093042

(56) References cited:
- WO-A1-2016/168846
- JP-B2- 5 721 905
- KR-A- 20100 010 714
- KR-A- 20100 056 882
- KR-A- 20120 015 227
- KR-A- 20160 056 707
- US-A1- 2011 208 031
- US-A1- 2013 120 748

## Description

### TECHNICAL FIELD

The present disclosure relates to a deep brain stimulation apparatus based on surface-enhanced Raman spectroscopy.

### BACKGROUND ART

In current deep brain stimulation systems, electrodes are inserted into a specific site of the brain that is highly related to the disease and receives electrical stimulation from a stimulator inserted in the human body to adjust excitation and inhibition of nerves related to the disease by electrical stimulation, and thus it exhibits a therapeutic effect on various brain diseases. For example, motor function of patients with Parkinson's disease or hypochondria may be restored through electrical stimulation. Recently, it has been applied not only to physical disorders but also to mental disorders such as chronic pain and depression.

A conventional deep brain stimulation system is an open loop brain stimulation system that continuously stimulates by using stimulation parameters (intensity, frequency, waveform, amplitude, etc.) set by a physician's clinical experience without considering changes in a patient's symptoms due to electrical stimulation.

Document WO 2016 168 846 discloses a deep brain stimulation electrode. The electrode may be covered with a molecular biofunctional layer; further, SERS sensors are mentioned.

However, such an open loop system consumes high power consumption due to continuous stimulation, which results in shortening of the life of a battery inserted into the human body. This increases the risk of surgical expense and infection as the patient needs frequent battery replacement surgery, and continuous electrical stimulation leads to addiction to the stimulus, which reduces the response to the stimulus, which accordingly causes a reduction in the therapeutic effect of the stimulus over time as well as other side effects.

Therefore, in order to improve the disadvantages of deep brain stimulation systems using open loop systems, there is a need for a closed-loop system that may actively control the symptoms of a patient that change over time by using a feedback biomarker that may represent a disease condition of a patient. In order to measure the brain response, conventionally, a method of inserting a microelectrode into the brain or chemically detecting a neurotransmitter has been used.

However, the method using the measurement electrode was not suitable for the following reasons. The deep brain stimulation apparatus should repeat stimulation and measurement for a long time. Conventionally used measurement electrodes deteriorate in performance with respect to electrodes which sense an electric signal due to cellular or fibrous encapsulation in which a conductive part of the electrode is wrapped around the brain tissue when the electrode is inserted into the brain tissue for a long period of time. Also, because a silicon-based monitoring electrode is not flexible, the electrode may be broken when inserted into the brain tissue or the brain tissue may be damaged. When the response of the brain tissue to the electrical stimulation is observed through the electrical signal, distortion of the signal due to the input electrical signal is inevitable and the accuracy of measurement is reduced.

Also, a chemical measurement method, that is, a voltammetry method, provides a quick and direct method of detection of neurotransmitters, but there is a problem in that it is difficult to detect a neurotransmitter having a trace concentration *in vivo* and detection efficiency deteriorates.

Also, in order to monitor changes in a neurotransmitter by stimulation in real time, there is a need for a technology capable of detecting a neurotransmitter present in a trace concentration with high sensitivity, with a fast temporal resolution of 100 ms, and with a spatial resolution of several tens *µ*m, but the existing technology has not reached this level.

Raman spectroscopy may be used to overcome this problem. Scattering is a phenomenon in which light passes through a medium and the wavelength of the light is changed such that a portion of the light travels in a different direction away from a traveling direction. The scattering phenomenon that changes the wavelength of light is called Raman scattering. When light passes through a medium, a portion of the light is scattered and travels in the different direction away from the traveling direction. At this time, the scattered light has the original energy as it is, but it may have less or more energy than the original energy of light. A process of scattering the scattered light while maintaining its original energy is called Rayleigh Scattering (elastic scattering), and a process of scattering the scattered light while losing or gaining energy is called Raman scattering (inelastic scattering).

When a molecule receives light energy such as ultraviolet light or visible light, the molecule is in an excited state, and the molecule in this state emits light while falling back to a ground state.

When the molecule falls to the ground state while emitting all of the energy of firstly incident light, light having the same frequency as that of the incident light is scattered and emitted. This case is the above-described Rayleigh scattering. On the other hand, Raman scattering is the case where the molecule returns to the ground state after absorbing or emitting as much as the vibrational energy of the molecule. At this time, an electronic state does not change, but a transition of a vibrational state occurs. The case where the molecule returns to the ground state after absorbing the vibration energy of the molecule is called a Stokes shift. At this time, because energy of the radiation is absorbed by the molecule, light of a lower energy than that of the incident light, that is, light of a longer wavelength, is scattered. On the other hand, the case where the molecule returns to the ground state after emitting the vibrational energy of the molecule is called an anti-Stokes shift. Because this is a state where the radiation gains energy from the molecule, light of a higher energy than that of the incident light, that is, light of a shorter wavelength, is scattered. Through this Raman scattering process, energy exchange occurs between an incident light source and a material.

The energy absorbed or emitted by materials is closely related to the molecular structure that constitutes each material, and the spectrum of light due to Raman scattering is inherent in each material, and thus analysis of the scattered light may infer the molecular structure of the material.

Raman spectroscopy has been used to study the vibrational structure of a molecule by measuring a vibration spectrum of the molecule, or to qualitatively and quantitatively analyze a material, but recently it has also been applied to studies for analysis of biochemical and morphological information of intracellular or extracellular biological tissues.

### DESCRIPTION OF EMBODIMENTS

### TECHNICAL PROBLEM

However, when a laser beam is irradiated to a tissue in a visible light region, fluorescence or luminescence occurs in addition to Rayleigh scattering or Raman scattering. In this case, because the intensity is very strong in comparison with Raman scattering and occurs in a region similar to a region where the Raman spectrum is measured, when the Raman spectrum is measured, it is measured together, and thus a pure Raman spectrum may not be obtained.

Also, it is difficult to apply the Raman spectrum to real-time monitoring because a spectral signal is very weak. A Raman spectral signal may be improved by increasing an output of laser that is an incident excitation light. However, the use of high power laser has limitations because it induces deformation of biomolecule.

The present disclosure provides a deep brain stimulation apparatus and method based on a surface-enhanced Raman spectroscopy.

### SOLUTION TO PROBLEM

A deep brain stimulation apparatus based on surface-enhanced Raman spectroscopy includes a stimulator configured to apply electrical stimulation to a brain; a light source configured to emit light to the brain; plasmonic nano-particles configured to allow surface-enhanced Raman scattering (SERS) by contacting a neurotransmitter when the brain secretes the neurotransmitter as a result of the electrical stimulation; a photodetector configured to, when the light incident from the light source is scattered by the neurotransmitter in contact with the plasmonic nano-particles, detect scattered light through an optical fiber; a signal processing analyzer configured to convert a light signal from the photodetector into an electric signal; and a controller configured to receive an analysis signal of the signal processing analyzer to control the stimulator.

The plasmonic nano-particles may include one or more selected from the group consisting of gold, silver, platinum, palladium, copper, tin, nickel, and aluminum.

The photodetector may include an optical fiber including a core and a cladding surrounding the core.

The plasmonic nano-particles may be arranged to surround a surface of the optical fiber.

The cladding and the plasmonic nano-particles may include the same metal.

The photodetector is inserted into the body.

The signal processing analyzer is further configured to analyze a surface-enhanced Raman spectroscopic spectrum of the scattered light.

The controller is inserted into the body.

The controller may include a wireless communicator and a wireless charger.

The controller is further configured to control the stimulator when the analysis signal of the signal processing analyzer satisfies a preset condition.

A brain deep stimulation method based on surface-enhanced Raman spectroscopy according to the present disclosure may include applying electrical stimulation to a brain; allowing plasmonic nano-particles capable of surface-enhanced Raman scattering to come in contact with a neurotransmitter secreted as a result of the electrical stimulation; exposing the brain to a light; when the light is scattered by the neurotransmitter in contact with the plasmonic nano-particles, detecting the scattered light; converting a scattered light signal into an electrical signal; analyzing the electrical signal; and controlling the electrical stimulation according to an analysis result of the electrical signal.

Additional aspects, features, and advantages other than those described above will become apparent from the accompanying drawings, the following claims, and the detailed description of the present disclosure.

### ADVANTAGEOUS EFFECTS OF DISCLOSURE

According to the deep brain stimulation apparatus and method based on surface-enhanced Raman spectroscopy according to the present disclosure, the Raman spectrum having a strong intensity may be obtained by a photodetector including plasmonic nano-particles capable of surface-enhanced Raman scattering without using a high output laser, and thus deformation of a biomolecule may be prevented.

In addition, in the deep brain stimulation system which applies electrical stimulation to a specific part of the brain for rehabilitation or therapeutic purposes, the neurotransmitter that is highly related to the disease may be monitored in real time and may be used to actively control a stimulation parameter applied to the brain, thereby providing feedback by reflecting a change of the neurotransmitter according to a symptom of the patient, and thus patient-customized treatment is possible.

### BRIEF DESCRIPTION OF DRAWINGS

FIG. 1 is a conceptual diagram of a deep brain stimulation apparatus based on surface-enhanced Raman spectroscopy, according to the present disclosure.
FIG. 2 is a conceptual diagram showing an example of plasmonic nano-particles.
FIG. 3 is a graph showing a surface-enhanced Raman spectrum of dopamine using plasmonic nano-particles.
FIGS. 4 and 5 are conceptual diagrams showing examples of a photodetector.
FIG. 6 is a conceptual diagram showing a configuration of a signal processing analyzer.
FIG. 7 is a conceptual diagram showing a configuration of a controller.
FIG. 8 is a flowchart showing an example of a control method performed by a controller.
FIG. 9 is a flowchart of a deep brain stimulation method based on surface-enhanced Raman spectroscopy, according to the present disclosure.

### BEST MODE

As the present disclosure allows for various changes and numerous embodiments, particular embodiments will be illustrated in the drawings and described in detail. Effects, features, and methods of achieving these of the present disclosure will become apparent from the following detailed embodiments with reference to the accompanying drawings. However, the present disclosure is not limited to the embodiments set forth herein, and may be embodied in many different forms.

Hereinafter, embodiments of the present disclosure will be described in detail with reference to the accompanying drawings, like reference numerals denote like or corresponding components throughout the drawings, and a detailed description thereof will be provided once.

An expression in the singular encompasses an expression in the plural unless it has a clearly different meaning in the context.

The terms such as including, having, and the like are intended to indicate the existence of the features or components described in the specification, and are not intended to preclude the possibility that one or more other features or components may be added.

In the drawings, the sizes of elements may be exaggerated or reduced for convenience of explanation. For example, the size and thickness of each element illustrated in the drawings are arbitrarily illustrated for convenience of explanation, and thus the present disclosure should not be limited to the illustrations of the drawings.

If an embodiment can be differently embodied, a specific step may be performed differently from the described order. For example, two successively described steps may be performed substantially concurrently, or may be performed in a reverse order to that described.

While the present disclosure has been described with reference to embodiments illustrated in the drawings, these embodiments are provided for illustrative purposes only and it will be understood by those of ordinary skill in the art that various changes and modifications are made therein. Therefore, the true scope of the present disclosure should by defined by the technical spirit of the appended claims.

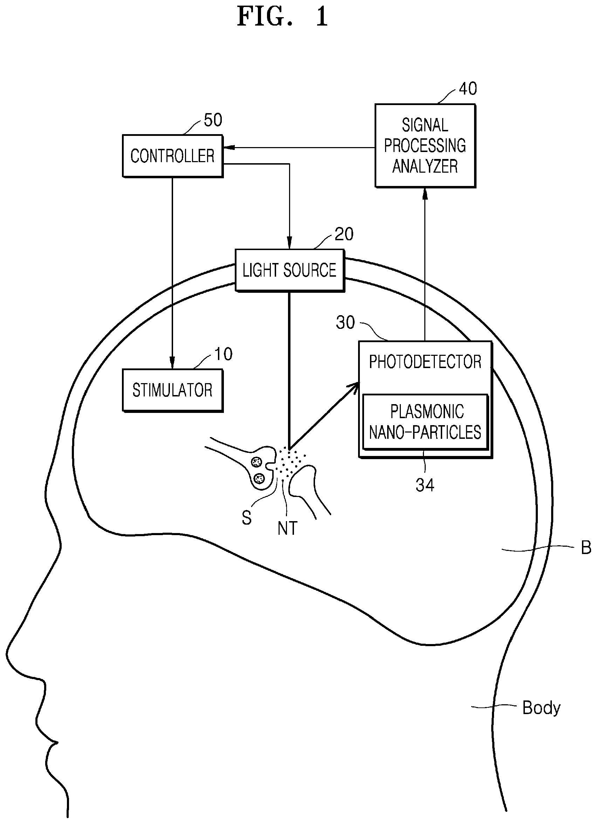

FIG. 1 is a conceptual diagram of a deep brain stimulation apparatus based on a surface-enhanced Raman spectroscopy according to the present disclosure.

The deep brain stimulation apparatus based on a surface-enhanced Raman spectroscopy according to the present disclosure includes a stimulator 10 that applies electrical stimulation to a brain B, a light source 20 that emits light to the brain B, plasmonic nano-particles 34 that allow surface-enhanced Raman scattering by contacting a neurotransmitter NT when the brain B secretes the neurotransmitter NT by the electrical stimulation, a photodetector 30 that includes the plasmonic nano-particles 34 and, when the light incident by the light source 20 is scattered by the neurotransmitter NT in contact with the plasmonic nano-particles 34, detects a scatted light, a signal processing analyzer 40 that converts a light signal from the photodetector 30 into an electric signal, and a controller 50 that receives an analysis signal of the signal processing analyzer 40 to control the stimulator 10.

The stimulator 10 stimulates the brain B to induce or suppress a specific reaction of the body. For example, when a hand shake, etc. occurs due to an abnormal neural circuit, it is possible to suppress the hand shake by applying a reverse voltage to a specific region of the brain B that induces the hand shake. That is, the reaction of the body due to the electrical stimulation of the stimulator 10 is observed by the light source 20 and the photodetector 30 that will be described later.

Although not shown, the stimulator 10 may include a communicator, an implanted pulse generator (IPG), a lead, and an extension. The communicator receives a control signal from the controller 50 that will be described later. The IPG generates electrical stimulation to induce or suppress specific neural activity by adjusting the magnitude, frequency, etc. of the stimulation signal according to the control signal of the controller 50. The lead transmits the electrical stimulation to a specific part of the brain B. The lead may include, for example, a polyurethane coil consisting of four platinum-iridium leads. A lead line is connected to the IPG through the extension. The stimulator 10 is inserted into the body by a surgical operation.

By the electrical stimulation, the neurotransmitter NT is secreted in a specific nerve path. That is, when the electrical stimulation is applied to the specific part of the brain B, a specific neural path matching therewith may be activated. Thus, by analyzing an amount of the neurotransmitter NT in the nerve path, effects of the electrical stimulation of the brain may be observed.

The light source 20 emits light to the brain B, in particular to, a synapse S of a specific nerve path due to electrical stimulation. The incident light is scattered by the neurotransmitter NT coupled to the plasmonic nano-particles 34 that will be described later and is detected by the photodetector 30.

The light emitted from the light source 20 may have a wavelength of, for example, 400 nm to 700 nm in a visible light region. For example, the light source 20 may use various lasers such as a He-Ne laser having a wavelength of 611.8 nm and 633.8 mm, an argon (Ar) laser having wavelengths of 454.6 nm, 488.0 nm, and 514.5 nm, a krypton (Kr) laser having wavelengths of 530.9 nm, 568.2 nm, 647.1 nm, and 676.4 nm, etc. The above wavelength band is suitable for causing surface plasmon resonance (SPR) of metal included in the plasmonic nano-particles 34. This will be described later.

When the light source 20 is disposed outside the body, it is necessary to increase the intensity of the incident light because the light must transmit through the brain tissue. In this case, noise in the photodetector 30 of the light source 20 is strong, but its installation is advantageously simple. Conversely, when the light source 20 is inserted into a nerve path corresponding to a specific region of the brain, in particular, the brain, a separate operation is required for insertion, but the detection sensitivity in the photodetector 30 is further improved.

### MODE OF DISCLOSURE

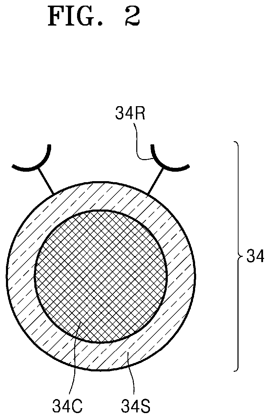

FIG. 2 is a conceptual diagram showing an example of the plasmonic nano-particles 34.

The deep brain stimulation apparatus based on a surface-enhanced Raman spectroscopy according to the present disclosure includes the plasmonic nano-particles 34 which allow surface-enhanced Raman scattering (SERS) by contacting the neurotransmitter NT when the brain B secretes the neurotransmitter NT by electrical stimulation.

According to an embodiment, the plasmonic nano-particles 34 may include one or more selected from the group including gold, silver, platinum, palladium, copper, tin, nickel, and aluminum.

A surface-enhanced Raman scattering (SERS) effect indicates a phenomenon that when a target substance is adsorbed on surfaces of nano-particles causing surface plasmon resonance (SPR) such as gold, silver, platinum, etc, the Raman scattering intensity increases about 10³ to 10¹⁴ times or more.

Surface plasmon resonance (SPR) is a resonance phenomenon that occurs when the frequency of light matches with natural frequencies of surface electrons oscillates with respect to the restoring force of a positive nucleus by the collective oscillation of electrons in a solid or liquid state stimulated by light. In particular, the surface plasmon resonance phenomenon in a nanometer-sized structure is called localized SPR. Plasmonic nano-particles, for example, substances adsorbed on metal surfaces, may be analyzed using the surface plasmon resonance phenomenon.

One of advantages of surface-enhanced Raman scattering (SERS) analysis is that the SERS may provide information that is difficult to obtain through general Raman analysis. In other words, because there are various surface interactions between the substance to be analyzed and the surface of the plasmonic nano-particles, an enhanced Raman signal which may not be obtained from the general Raman spectrum may be observed.

In order to increase the intensity of the Raman scattered light, a phenomenon that free electrons on the surface of the plasmonic nano-particles vibrate collectively between the plasmonic nano-particles and the incident light must be present, which is referred to as surface plasmon that is a basis of the electromagnetic enhancement effect. The incident light generates surface plasmon on the surface of the plasmonic nano-particles, and the surface plasmon increases the intensity of the Raman scattered light through the interaction with an analyte.

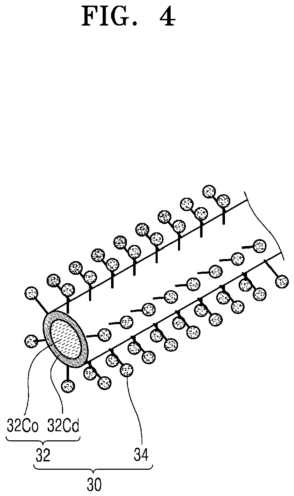

The plasmonic nano-particles 34 of the present disclosure have a structure that enables a surface-enhanced Raman signal. On the other hand, although it is expressed as a particle, the plasmonic nano-particles 34 do not necessarily have to be molecularly separated from each other, but the nano-particles 34 may mean poles, irregularities, etc., in nanometers (nm) protruding from a certain surface (e.g., an optical fiber (FIG. 4)) that will be described later.

According to an embodiment of FIG. 2, the plasmonic nano-particles 34 may include a nano-core 34C and a shell 34S surrounding the nano-core 34C.

The nano-core 34C may be modified in various ways if it has physical synthesis such as metal and silica as well as natural polymers such as keratin, collagen, gelatin, cellulose, chitosan, and synthetic polymer such as polymethyl methacrylate (PMMA).

The shell 34S may include at least one selected from the group including gold, silver, platinum, palladium, copper, tin, nickel, and aluminum, but is not limited thereto and may be a material capable of exhibiting the surface plasmon resonance phenomenon. This shell 34S contacts the neurotransmitter NT to amplify the Raman signal. The shell 34S may be coated on the surface of the nano-core 34C by, for example, electroless plating or the like, and may be in the range of 10 nm to 100 nm.

On the other hand, the plasmonic nano-particles 34 may include a receptor 34R for binding to the surface of the plasmonic nano-particles 34 with the neurotransmitter NT that is a target substance. The receptor 34R may include a target substance specific receptor consisting of any one selected from the group consisting of an enzyme substrate, a ligand, an amino acid, a peptide, a protein, a nucleic acid, a lipid, a carbohydrate or an antibody.

At this time, the target substance of the receptor 34R is not only the neurotransmitter NT that is cathecholamines dopamine, norepinephrine, fepretone, but also indoamines serotonin, melatonin and norepinephrine, serotonin , melatonin, phenylethylamine, tilamine, tryptamine, octopamine, gammaaminobutyric acid (GABA). Previously, electrochemical detection using an oxidation-reduction reaction was mainly used to detect neurotransmitters. Therefore, only catecholamine-based neurotransmitters that may be easily oxidized may be detected. However, according to the present disclosure using an optical method, various neurotransmitters NT may be detected without being limited thereto.

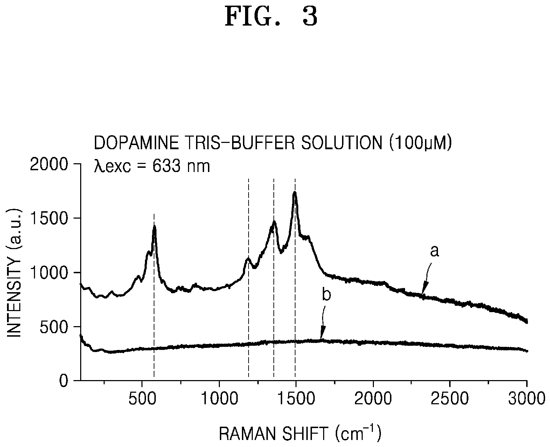

FIG. 3 is a surface-enhanced Raman spectrum graph of dopamine using the plasmonic nano-particles 34.

Dopamine is a representative neurotransmitter secreted by the brain. Dopamine deficiency causes various neurological diseases. In particular, dopamine concentration in patients with Parkinson's disease may be used as a feedback biomarker to represent the state of disease. The embodiment of FIG. 3 is the graph in which dopamine in a tris-buffer solution at a concentration of 100 uM is detected using the plasmonic nano-particles 34. In the graph, "a" represents the case where the plasmonic nano-particles 34 are added, and "b" represents the case where the plasmonic nano-particles 34 are not added. A peak position and an intensity of a Raman shift peak of the "a" graph may be analyzed to estimate the presence and concentration of a detection substance dopamine. That is, in the existing method that does not use surface-enhanced Raman spectroscopy, it was impossible to measure dopamine, but dopamine measurement was possible through the surface-enhanced Raman spectroscopy using the plasmonic nano-particles (34).

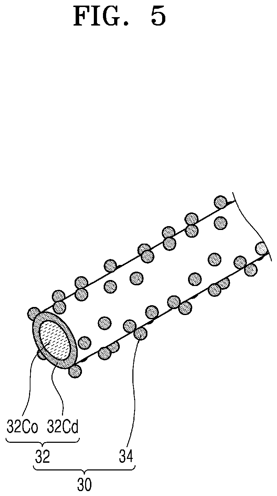

FIGS. 4 and 5 are conceptual diagrams showing examples of the photodetector 30. The photodetector 30 detects scattered light when a light incident on the light source 20 is scattered by the neurotransmitter NT contacting the plasmonic nano-particles 34. The photodetector 30 includes the plasmonic nano-particles 34.

According to an embodiment, the photodetector 30 may include an optical fiber 32.

According to the embodiment of FIG. 4, at this time the plasmonic nano-particles 34 may be arranged to surround the surface of the optical fiber 32. That is, the photodetector 30 includes the optical fiber 32 and the plasmonic nano-particles 34 surrounding the optical fiber 32. The optical fiber 32 is a thin and elongated fiber that transmits a light signal.

At this time, the surface of the optical fiber 32 may be modified such that the plasmonic nano-particles 34 and the optical fiber 32 are chemically bonded. For example, the surface of the optical fiber 32 may be surface-modified to have a hydrophilic property. In this case, when the nano-core 34C of the plasmonic nano-particles 34 is made of a hydrophilic substance, the plasmonic nano-particles 34 and the optical fiber 32 may be bonded.

According to the embodiment of FIG. 5, a cladding 32Cd of the optical fiber 32 and the plasmonic nano-particles 34 may include the same metal. The cladding 32Cd itself and metal of the plasmonic nano-particles 34 may be deposited to a core 32Co of the optical fiber 32 differently from the case of FIG. 4 in which the surface modification is performed separately for bonding the optical fiber 32 and the plasmonic nano-particles 34. At this time, the surface of the cladding 32Cd has a roughness in nanometer (nm) due to the plasmonic nano-particles 34 that protrude, and thus the plasmon resonance phenomenon is more likely to occur.

According to an embodiment, the photodetector 30 may be inserted into the body.

As described above, when the plasmonic nano-particles 34 and the neurotransmitter NT are in contact with or bonded to each other, when light emitted from the light source 20 is scattered in the neurotransmitter NT, because the intensity of surface-enhanced Raman scattering is greatly enhanced, the scattered light may be detected by the photodetector 30. That is, in order to obtain the surface-enhanced Raman scattering effect, the plasmonic nano-particles 34 of the photodetector 30 may be inserted into the body and contacted with the neurotransmitter NT propagating between the synapses S of a nerve path to be measured. In addition, when the scattered light exits outside of the body, because the intensity thereof is rapidly reduced, the photodetector 30 may be inserted into the body, in particular, the brain B, and near the scattered light, and near the synapses S of the nerve path to be measured. (See FIG. 1)

When the plasmonic nano-particles 34 include the receptor 34R, the neurotransmitter NT may be chemically bonded to the receptor 34R. At this time, the light incident on the light source (20 of FIG. 1) generates surface plasmon on the surface of the plasmonic nano-particles 34, and the surface plasmon increases the intensity of Raman scattered light through the interaction with the neurotransmitter NT. The Raman scattered light is reflected by the tissue near the synapse or the like, or enters the core 32Co of the optical fiber 32 through the cladding 32Cd. The scattered light is totally reflected on the interface between the core 32Co and the cladding 32Cd and travels in the core 32Co. The signal processing analyzer (40 of FIG. 1) is disposed at the opposite end of the optical fiber 32 to sense light.

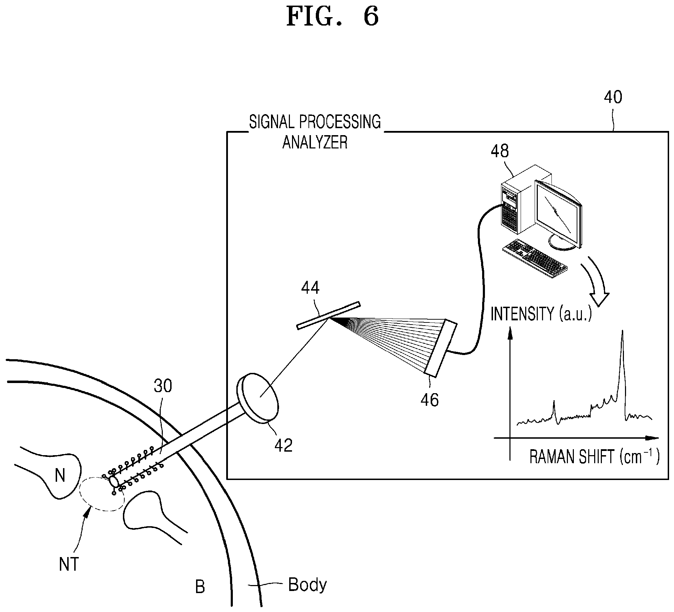

FIG. 6 is a conceptual diagram showing a configuration of the signal processing analyzer 40.

The signal processing analyzer 40 converts a light signal from the photodetector 30, that is, a surface-enhanced Raman scattered light, into an electric signal and analyzes the electric signal. The signal processing analyzer 40 may include a filter 42 for filtering a specific wavelength of light detected by the photodetector 30, a grating 44 for dividing the light passing through the filter 42 by wavelength, and a charge coupled device (CCD) 46 for converting the signal into the electrical signal. When the light signal is converted into the electric signal by the CCD 46, the electric signal is analyzed through a computer 48 or the like to obtain a Raman spectrum. The signal processing analyzer 40 obtains information about a type and concentration of the neurotransmitter NT, etc. from the Raman spectrum.

The signal processing analyzer 40 may be disposed outside the body. In this case, for example, the photodetector 30 inserted into the body may have a protruding portion outside the body, and may transmit the light signal to the signal processing analyzer 40. When the signal processing analyzer 40 is inserted into the body, it is preferable that the signal processing analyzer 40 is manufactured to be very compact and enables wireless communication and wireless charging.

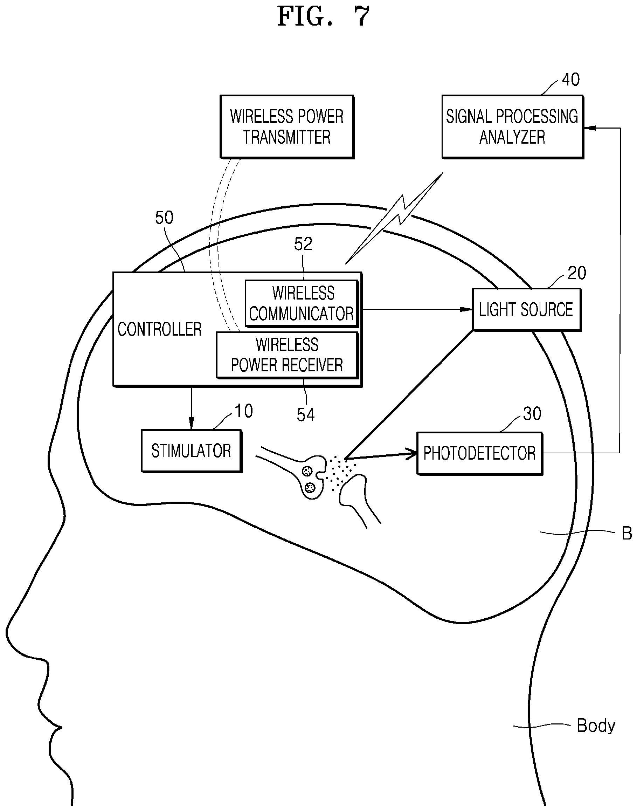

FIG. 7 is a conceptual diagram showing a configuration of the controller 50. Referring to FIG. 7, the controller 50 receives a signal from the signal processing analyzer 40 and analyzes the signal to control the stimulator 10. Meanwhile, the controller 50 may control the intensity of the light source 20, a frequency, and the like.

According to an embodiment, the controller 50 may be inserted into the body.

According to an embodiment, the controller 50 may include a wireless communicator 52 and a wireless power receiver 54. Referring to FIG. 7, the wireless communicator 52 wirelessly transmits and receives signals to and from the signal processing analyzer 40. The wireless power receiver 54 receives power from an external wireless power transmitter through a magnetic resonance principle. In this case, there is an advantage that a separate operation for replacing a battery of the controller 50 is not necessary.

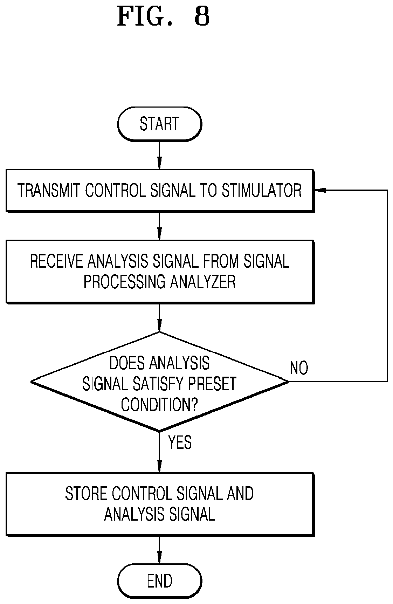

FIG. 8 is a flowchart showing an example of a control method performed by the controller 50.

According to an embodiment, the controller 50 may control the stimulator 10 when an analysis signal of the signal processing analyzer 40 satisfies a preset condition.

Referring to FIG. 8, the controller 50 first transmits a control signal to the stimulator 10. For example, the control signal may be for electrically stimulating a specific part of the brain to mitigate a handshake of a patient. At this time, electrical stimulation parameters (intensity, frequency, waveform, amplitude, etc.) according to a first control signal may be set by physician's clinical/statistical experience.

Thereafter, the controller 50 receives the signal analyzed by the signal processing analyzer 40. For example, the analysis signal may include concentration information of the neurotransmitter N) estimated from the intensity of the Raman spectrum.

Thereafter, the controller 50 determines whether the analysis signal satisfies a preset condition. For example, when there is no change in the concentration of the neurotransmitter NT in a neural circuit that causes a handshake even though an electrical stimulation signal is applied in order to reduce a handshake phenomenon or an amount of change is smaller than a threshold value, the controller 50 determines that the analysis signal does not satisfy the preset condition and transmits a control signal to apply a larger electrical stimulation to the stimulator 10. If the concentration of neurotransmitter NT in the neural circuit that causes the handshake by the electric stimulus signal to reduce the handshake phenomenon decreases, the controller 50 determines that the analysis signal does not satisfy the preset condition and store the control signal and the analysis signal. Thereafter, the controller 50 transmits the control signal to the stimulator 10 based on the stored information.

On the other hand, the preset condition may be not only the above-described condition but also, for example, whether the concentration change rate of the neurotransmitter NT with respect to the intensity of the electrical stimulation is equal to or greater than or smaller than or equal to the threshold value.

That is, the controller 50 controls the stimulator 10 by feeding back the analysis signal received from the signal processing analyzer 40. Specifically, when the controller 50 transmits the control signal to the stimulator 10 and the stimulator 10 stimulates a specific part of the brain, accordingly a neural circuit related to a specific movement /accident according to the electrical stimulation reacts. The reaction detected by the photodetector 30 is analyzed by the signal processing analyzer 40 and sent to the controller 50. The controller 50 feeds back the reaction to transmit the control signal to the stimulator 10 again. That is, the stimulator 10 calculates, stores, and analyzes the correlation between the electrical stimulation and the nerve circuit reaction. Accordingly, it is possible to set the optimal stimulation condition for a patient by actively adjusting an electrical stimulation parameter by feeding back the change of the neurotransmitter NT according to the patient's symptoms.

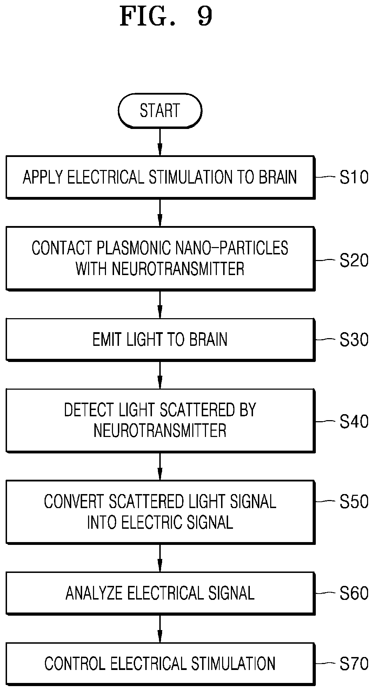

FIG. 9 is a flowchart of a deep brain stimulation method based on a surface-enhanced Raman spectroscopy according to the present disclosure.

Referring to FIGS. 1 and 9, the deep brain stimulation method based on the surface-enhanced Raman spectroscopy according to the present disclosure includes step S10 of applying electrical stimulation to the brain B, step S20 of contacting the plasmonic nano-particles 34 which allow surface-enhanced Raman scattering with the neurotransmitter NT secreted by the electrical stimulation, step S30 of emitting light to the brain B, step S40 of, when the light is scattered by the neurotransmitter NT in contact with the plasmonic nano-particles 34, detecting a scatted light, step S50 of converting a scattered light signal into an electric signal, step S60 of analyzing the electrical signal, and step S70 of controlling the electrical stimulation according to an analysis result of the electrical signal.

At this time, although step S10 of applying the electrical stimulation to the brain B, step S20 of contacting the plasmonic nano-particles 34 with the neurotransmitter NT, and step S30 of emitting light to the brain B are sequentially performed in FIG. 9, the present disclosure is not limited thereto. That is steps S10, S20, and S30 may be performed substantially simultaneously, and step S20 of contacting the plasmonic nano-particles 34 with the neurotransmitter NT may be performed before step S10 of applying the electrical stimulation to the brain B.

According to the deep brain stimulation apparatus and method based on a surface-enhanced Raman spectroscopy according to the present disclosure, the Raman spectrum having a strong intensity may be obtained by a photodetector including a plasmonic nano-particle capable of surface-enhanced Raman scattering without using a high output laser, and thus deformation of a biomolecule may be prevented.

In addition, in the deep brain stimulation system which applies electrical stimulation to a specific part of the brain for rehabilitation or therapeutic purposes, the neurotransmitter that is highly related to the disease may be monitored in real time and may be used to actively control a stimulation parameter applied to the brain, thereby providing feedback by reflecting a change of the neurotransmitter according to a symptom of the patient, and thus the patient customizing treatment is possible.

The invention is defined in the following claims. Other examples as well as methods do not constitute parts of the invention.

### INDUSTRIAL APPLICABILITY

The present disclosure relates to a brain deep stimulation apparatus based on the surface-enhanced Raman spectroscopy and can be used in the diagnostic and therapeutic apparatus industry.

## Claims

1. A deep brain stimulation apparatus based on surface-enhanced Raman spectroscopy, the deep brain stimulation apparatus comprising: a stimulator (10) configured to apply electrical stimulation to a brain;
**characterised by** further comprising:
a light source (20) configured to emit light to the brain; plasmonic nano-particles (34) configured to allow surface-enhanced Raman scattering, SERS, by contacting a neurotransmitter when the brain secretes the neurotransmitter as a result of the electrical stimulation;
a photodetector (30) configured to, when the light incident from the light source is scattered by the neurotransmitter in contact with the plasmonic nano-particles, detect scattered light through an optical fiber;
a signal processing analyzer (40) configured to convert a light signal from the photodetector into an electric signal; and
a controller (50) configured to receive an analysis signal of the signal processing analyzer to control the stimulator,
wherein the plasmonic nano-particles are bonded to a surface of the optical fiber and inserted into a body together with the optical fiber.

2. The deep brain stimulation apparatus of claim 1, wherein the plasmonic nano-particles comprise one or more selected from the group consisting of gold, silver, platinum, palladium, copper, tin, nickel, and aluminum.

3. The deep brain stimulation apparatus of claim 1, wherein the photodetector is insertable into the body.

4. The deep brain stimulation apparatus of claim 1, wherein the signal processing analyzer is further configured to analyze a surface-enhanced Raman spectroscopic spectrum of the scattered light.

5. The deep brain stimulation apparatus of claim 1, wherein the controller is insertable into the body.

6. The deep brain stimulation apparatus of claim 1,
wherein the controller comprises a wireless communicator and a wireless charger.

7. The deep brain stimulation apparatus of claim 1,
wherein the controller is further configured to control the stimulator when the analysis signal of the signal processing analyzer satisfies a preset condition.

8. The deep brain stimulation apparatus of claim 1,
wherein a surface of the optical fiber is modified to have a hydrophilic property.

## Patentansprüche

1. Einrichtung für die tiefe Hirnstimulation, die auf einer oberflächenverstärkten Raman-Spektroskopie basiert, wobei die Einrichtung für die tiefe Hirnstimulation umfasst:
einen Stimulator (10), der dazu konfiguriert ist, eine Elektrostimulation an ein Gehirn anzulegen;
**dadurch gekennzeichnet, dass** sie weiter umfasst:
eine Lichtquelle (20), die dazu konfiguriert ist, Licht auf das Gehirn zu emittieren;
plasmonische Nanopartikel (34), die dazu konfiguriert sind, durch Kontaktieren eines Neurotransmitters eine oberflächenverstärkte Raman-Streuung, SERS, zu ermöglichen, wenn das Gehirn den Neurotransmitter als eine Folge der elektrischen Stimulation sekretiert;
einen Fotodetektor (30), der dazu konfiguriert ist, gestreutes Licht durch einen Lichtwellenleiter zu detektieren, wenn das von der Lichtquelle einfallende Licht durch den Neurotransmitter, der mit den plasmonischen Nanopartikeln in Kontakt ist, gestreut wird;
eine Signalverarbeitungsanalyseeinheit (40), die dazu konfiguriert ist, ein Lichtsignal von dem Fotodetektor in ein elektrisches Signal umzuwandeln; und
eine Steuereinheit (50), die dazu konfiguriert ist, ein Analysesignal der Signalverarbeitungsanalyseeinheit zu empfangen, um den Stimulator zu steuern,
wobei die plasmonischen Nanopartikel an eine Oberfläche des Lichtwellenleiters gebunden sind und zusammen mit dem Lichtwellenleiter in einen Körper eingesetzt sind.

2. Einrichtung für die tiefe Hirnstimulation nach Anspruch 1,
wobei die plasmonischen Nanopartikel eines oder mehrere umfassen, die aus der Gruppe ausgewählt sind, die aus Gold, Silber, Platin, Palladium, Kupfer, Zinn, Nickel und Aluminium besteht.

3. Einrichtung für die tiefe Hirnstimulation nach Anspruch 1,
wobei der Fotodetektor in den Körper einsetzbar ist.

4. Einrichtung für die tiefe Hirnstimulation nach Anspruch 1,
wobei die Signalverarbeitungsanalyseeinheit weiter dazu konfiguriert ist, ein Oberflächenverstärkte-Raman-Spektroskopie-Spektrum des gestreuten Lichts zu analysieren.

5. Einrichtung für die tiefe Hirnstimulation nach Anspruch 1,
wobei die Steuereinheit in den Körper einsetzbar ist.

6. Einrichtung für die tiefe Hirnstimulation nach Anspruch 1,
wobei die Steuereinheit einen drahtlosen Kommunikator und ein drahtloses Ladegerät umfasst.

7. Einrichtung für die tiefe Hirnstimulation nach Anspruch 1,
wobei die Steuereinheit weiter dazu konfiguriert ist, den Stimulator zu steuern, wenn das Analysesignal der Signalverarbeitungsanalyseeinheit eine vorgegebene Bedingung erfüllt.

8. Einrichtung für die tiefe Hirnstimulation nach Anspruch 1,
wobei eine Oberfläche des Lichtwellenleiters modifiziert ist, um eine hydrophile Eigenschaft aufzuweisen.

## Revendications

1. Appareil de stimulation cérébrale profonde basée sur une spectroscopie Raman exaltée de surface, l'appareil de stimulation cérébrale profonde comprenant : un stimulateur (10) configuré pour appliquer une stimulation électrique à un cerveau ;
**caractérisé par le fait qu'**il comprend en outre :
une source de lumière (20) configurée pour émettre de la lumière vers le cerveau ;
des nanoparticules plasmoniques (34) configurées pour permettre une diffusion Raman exaltée de surface, SERS, en entrant en contact avec un neurotransmetteur lorsque le cerveau sécrète le neurotransmetteur à la suite de la stimulation électrique ;
un photodétecteur (30) configuré pour, lorsque la lumière incidente provenant de la source de lumière est diffusée par le neurotransmetteur en contact avec les nanoparticules plasmoniques, détecter la lumière diffusée à travers une fibre optique ;
un analyseur de traitement de signal (40) configuré pour convertir un signal lumineux provenant du photodétecteur en un signal électrique ; et
un dispositif de commande (50) configuré pour recevoir un signal d'analyse de l'analyseur de traitement de signal pour commander le stimulateur,
dans lequel les nanoparticules plasmoniques sont liées à une surface de la fibre optique et insérées dans un corps avec la fibre optique.

2. Appareil de stimulation cérébrale profonde selon la revendication 1,
dans lequel les nanoparticules plasmoniques comprennent un ou plusieurs sélectionnés à partir du groupe consistant en de l'or, de l'argent, du platine, du palladium, du cuivre, de l'étain, du nickel et de l'aluminium.

3. Appareil de stimulation cérébrale profonde selon la revendication 1,
dans lequel le photodétecteur peut être inséré dans le corps.

4. Appareil de stimulation cérébrale profonde selon la revendication 1,
dans lequel l'analyseur de traitement de signal est en outre configuré pour analyser un spectre spectroscopique Raman exalté de surface de la lumière diffusée.

5. Appareil de stimulation cérébrale profonde selon la revendication 1,
dans lequel le dispositif de commande peut être inséré dans le corps.

6. Appareil de stimulation cérébrale profonde selon la revendication 1,
dans lequel le dispositif de commande comprend un communicateur sans fil et un chargeur sans fil.

7. Appareil de stimulation cérébrale profonde selon la revendication 1,
dans lequel le dispositif de commande est en outre configuré pour commander le stimulateur lorsque le signal d'analyse de l'analyseur de traitement de signal satisfait une condition prédéfinie.

8. Appareil de stimulation cérébrale profonde selon la revendication 1,
dans lequel une surface de la fibre optique est modifiée pour présenter une propriété hydrophile.
